# EUROPEAN PATENT APPLICATION

(11) **EP 0 663 448 A1**
(43) Date of publication of application: **19.07.1995**
(21) Application number: 94500206.1
(22) Date of filing: 20.12.1994
(51) Int. Cl.: C12Q 1/68, C12Q 1/70, G01N 33/569

(54) **Process for structurally analyzing the genome of viral and subviral pathogens**

(30) Priority: 21.12.1993 ES 9302657
(71) Applicant: INSTITUTO NACIONAL DE INVESTIGACION Y TECNOLOGIA, AGRARIA Y ALIMENTARIA, E-28003 Madrid (ES)
(72) Inventor: Nuno Barbosa, Gustavo, (Gambelas) 8000 Faro (PT); Martinez, David, ES-28224 Pozuelo de Alarcon, (Madrid) (ES); Sanchez, Flora, ES-28008 Madrid (ES); Romero, Javier, ES-28230 Las Rozas, (Madrid) (ES); Ponz, Fernando, ES-28230 Las Rozas, (Madrid) (ES); Torres Pascual, Vicente, ES-28029 Madrid (ES)
(74) Representative: Gonzalez Vacas, Eleuterio

(57) **Abstract**

The present invention refers to a process for structurally analyzing the genome of viral and subviral pathogens. The process starts up with the immobilization of macromolecular structures solid phase related with the pathogen. After the pathogen immobilization the enzymatic amplification of a fragment of its genome is carried out in the case of pathogens with ADN genome. When there is the case of pathogens with RNA genome, after the immobilization and before the enzymatic amplification, the inverse transcription of a fragment of the pathogen genome is carried out. The process ends with a study of the structure of the resulting products in the enzymatic amplification phase.

## Description

### BACKGROUND OF THE INVENTION

The analysis of the genome structure of viral and subviral pathogens produces very valuable information to use in the diagnosis and molecular epidemiology, in genes searching programs which confer resistance to such pathogens and in epitopes design projects for the production of new vaccines. In all the cases it is necessary to process without ambiguity and at low cost a high number of samples during short periods of time. It is desirable that the processes used would be sensitive, precise, specific, fast and that do not require installations, equipment and materials neither complex nor expensive as well as operators with high technical training, (Matthews, R.E.F. (1991). Methods for Assay, Detection and Diagnosis. In Plant Virology 3th Edition, pp 11. Academic Press Inc. New York).

The genome structural study of a viral or subviral pathogen by means of conventional processes starts up with the isolation of the pathogen from infected tissues of the host. This phase may become limiting in many occasions due to the low performance or to the deficient quality of the isolate. The pathogen low infection titration, the instability of its particles, and the nature of the starting material are some of the main factors which condition the performance and the quality of the isolates of viral and subviral pathogens. The performance obtained in the purification of viral and subviral pathogens which infect tissues or organs at very low titration, may be insufficient to carry out the genome structural analysis. The examples are vegetable viruses limited to phloem (Geminivirus, Luteovirus or Closterovirus) or some retrovirus like HIV (AIDS) or other limited to an organ or tissue (hepatitis, virus of nervous tissue, etc...). The structural stability of the particles of some viral pathogens may condition their purification. Some classic example are the cucumber mosaic virus (CMV), the Closterovirus and the Furovirus; their virions are so unstable that their purification is a very difficult task. The nature of the starting material for the isolation of the pathogen may represent a serious limitation in the production of isolates with the performance and sufficient quality to continue with the structural characterization of the successive phases of the pathogen genome, it is the case of tissues and organs rich in polyphenols or starch. On the other hand, the isolation of viral and subviral pathogens by means of conventional processes is an intrinsically tedious process and therefore very inadequate for the routine use.

The phase following purification of the viral and subviral pathogen in a study of the structure of its genome consists in the increase of the number of copies of the whole mentioned genome or of fragments thereof, traditionally produced by means of molecular clonation. Similarly to the case of purification the molecular clonation is a process intrinsically tedious, specially when it is related to viral pathogens with RNA genome or subviral pathogens, where the previous synthesis of the complementary (cADN) is required. Since the appearance of the ADN enzymatic amplification technology through the chain reaction of the polymerase (PCR) (Elrich, H.S. et al. E.P. 258.017; Cohen, S.N.U.S. Patent N° 4.293.652; Mullis, K. B. et al, Meth. Enzymol 155: 335-350 (1987); Scharf, R.K. et al., Science 233: 1076-1079 (1986), an alternative is offered to increase the number of copies of a viral genome fragment without requiring molecular clonation thereof. Also this technique has permitted to make the structural analysis of the complete genome of a Hantavirus without previous purification (Nichol, S.T. et al, 1991, Science 262:914-917) and also has been used with success for differentiating virus strains immunologically undistinguishable **(Patent GB 2252323).**

The polymerase chain reaction (PCR) is a very specific and efficient method, theoretically capable of synthesizing more than one million copies of a single sequence of an ADN mold. This technique has been used in the identification of viral pathogens, for which purpose the amplification of specific sequences of their genomes is carried out (Rybicky, E.P. and Hughes, F.L. (1990). J. Gen Virol 71: 2519-2526; Pasamontes et al (1991) J. of Virol Meth. 35: 143-147). For the identification of viral pathogens with RNA genome, there are methods described which combine the synthesis of complementary ADN (cADN) to the viral RNA and its later amplification through the polymerase chain reaction (RT-PCR), such methods have been applied for the identification of the different viruses, either in animals (Lin. S.T. et al. (1991). J. of Virol Meth. 35:227-236; Meyer, R.F. et al. (1991). J. of Virol Meth. 34:161-172) or in vegetables (Vunish, R. et al) (1990). Ann. Appl. Biol. 117: 561-569; Korschineek et al (1991). J. Virol Meth. 31: 139-146; Borja; M.J. and Ponz, F. (1992). J. Virol Meth., 36: 73-86). Although these methods are very sensitive and specific, they require excessive manipulation and the use of some chemical regents as phenol, highly toxic, therefore not being ideal for using as a routine Wetzel, T. et al. (1991). J. of Virol. Meth. 33: 355-365 and Borja, M.J. and Ponz, F. (1992). J. of Virol Meth. 36: 73-86, have described respectively the genome fragments amplification of the sharka virus and of the walnut strain of the cherry winding virus (wCLRV) in homogenates of tissue by means of RT-PCR without previously phenolized; nevertheless these methods do not have a general application character to identify other vegetable virus, probably due to the presence of inhibitors depending on the nature of the starting material.

Some methods have been described which pretend to combine the sensitivity and specificity of the PCR with a purification of the pathogen through its immobilization on a solid support. Thus, Jansen, R.W. et al (1990), Procc. Nat. Acad. Sci. USA 87: 2867-2871 and Wetzel, T. et al., (1992) . J. Virol. Methods 39, 355-365, describing respectively the detection of the hepatitis A virus and of the sharka virus by RT-PCR introducing a purification phase of the virus through antibodies retained on the walls of a eppendorf tube, followed by the thermic rupture of the virion previous to the RT and enzymatic amplification. Liang. T. and Wands, J.R. (1988). **(U.S. Patent 262347)** describe a virus detection method of the hepatitis B virus which combines the capture of the pathogen using antibodies linked to activated Sepharose with cyanogen bromide with the enzymatic amplification of the viral ADN and the electrophoretic identification of the products of amplification. None of these methods are of general application and all of them continue being too arduous and therefore not adequate to process a high number of samples.

We have described a process for the detection and identification of viral and subviral pathogens that combining the immobilization of the pathogen on a solid phase with the enzymatic amplification of a fragment of their genome permits the production of a high number of copies of such fragment neither requiring purification of the pathogen nor molecular clonation. Such process is sensitive, specific, simple and of general application, being for all these reasons applicable in a routine way **(Nolasco, G. et al 1992. p 9201232).**

The genome structural study of a viral or subviral pathogen ends with the analysis of the structural characteristics of the fragments of such amplified genomes. To carry out such analysis by conventional methods results too complex for routine use, since certain technologies are needed which require sophisticated instrumentation and a high technical preparation of the analyst. Molecular Biology has supply a group of techniques of easy execution which provide a valuable information on the structure of the nucleic acid. Some of these techniques; as the forming of sequences with nucleic acids (Maxam, A.M. and Gilbert, W. 1977.Proc. Natl. Acad. Sci. 74:560 and Sanger, F. et al., 1977. Proc. Natl. Acad. sci. 74:5463) and the analysis of restriction fragment length polymorphism (RFLP) (Tanskley, S.D. et al., 1989. Biotechnology 7:253 and Patent DE 4209216) or of single sequence conformation (SSCP) (Orita, M. et al., 1989. Genomics 5, 874-879), Patent US 7751892 and Patent DE 4209216; have been widely used in the genome structural analysis of different species.

### OBJECT OF THE INVENTION

The present invention refers to a new process for structural analysis of the genome of viral and subviral pathogens. The process combines the immobilization on a solid phase of macromolecular structures related to the pathogens, the enzymatic amplification of genomes fragments and the structural analysis of the resulting products of such amplification.

### DESCRIPTION OF THE INVENTION

The process to structurally analyze the genome of viral and subviral pathogens, which the invention proposes, comprises the following phases:
A) Immobilization on a solid phase of macromolecular structures related to the pathogen, directly carried out and/or by means of molecules, with affinity for such macromolecular structures, retained on such solid phase.
B) Synthesis of complementary ADN (cADN) to a fragment of the pathogen genome.
C) Enzymatic amplification of the cADN or of a fragment of ADN virus genome.
D) Analysis of the structural polymorphisms existing in the reaction products resulting from the enzymatic amplification.

The process which the invention proposes is a very useful tool to carry out structural studies of the genome of viral and subviral pathogens in a simple, fast, general way and with high sensitivity and specificity. Also, it is susceptible of automatization, which allows to apply it as routine in programs such as epidemiology or molecular typification of viral and subviral pathogens and in designing epitopes or vaccines, where it is required to obtain molecular information of a very high number of samples.

The immobilization of the pathogen on a solid phase and the later amplification of a fragment of its genome, eliminate the purification phases of the pathogen and molecular clonation of the fragment, necessary, up to the present time, for studies at molecular titration of the pathogen. These phases, traditionally essential for carrying out molecular studies are tedious, significantly make more expensive the process and also require complex equipment and highly qualified personnel, characteristics that have limited the use of such studies in programs where the analysis of a high number of samples is necessary.

With the process object of the present invention and through the use of molecules retained on a solid phase and which present specific affinity for molecular structures related to the pathogen, in this way the specific immobilization of such structures is achieved. If besides a microtitrating plate is used as solid phase, it is possible to carry out structural routine studies of the pathogen, such as the establishment of the nucleotides sequence, the analysis of restriction polymorphisms or the analysis of single chain conforming polymorphisms, applying the obtained results to the molecular typification of different viral and subviral pathogens.

The process object of the present invention is of general application, since through it we have been able to typify at molecular level, starting from biological samples of different nature and origins, viral pathogens with biological and architectural characteristics so different as the ones produced by the families of the Tobamovirus, Potyvirus, Closterovirus, Luteovirus, Nepovirus, Cucumovirus and Tospovirus.

The embodiment of the process object of the present invention using preferably antibodies against RNAs of double chain to immobilize on a solid phase molecular structures related to the pathogen, may be applied for the molecular typification of subviral pathogens and in the case of those viral pathogens for which there are no antibodies available against encoded proteins by their genome. If besides the previous embodiment is combined with the preferential use of a microtitrating plate as solid phase to carry out the immobilization, the process is susceptible of automatization, which permits using it in a routine manner to carry out the molecular studies of subviral pathogens and of those viruses for which there are no available specific antibodies.

The recommended process in the present invention is highly sensitive, which is a common characteristic of the methods based in enzymatic amplification. This high sensitivity permits that the process object of the present invention may be applied to the structural study of viral and subviral pathogens in those situations in which such pathogens are in low titration, some examples of those are the initial states of infection, vegetable viral and subviral pathogens transmitted by vegetative forms, vegetable viral pathogens limited to phloem as the Closterovirus or the animal viral pathogens limited to tissue or organ. The high sensitivity of the present process, combined with a preferential embodiment thereof in which the solid phase on which the macromolecular structures are immobilized related to the pathogen is a microtitrating plate, they form a system of great usefulness to carry out in a routine manner structural studies of viral and subviral pathogens when their titration is low.

The specificity of the present process, is given by the nucleotides sequence of the feeders used in the inverse transcription phases and enzymatic amplification. Such specificity is increased in the case of a preferential embodiment of the process in which specific antibodies are used against encoded proteins by the pathogen genome, retained on a solid phase, for the immobilization upon such support of macromolecular structures related to the pathogen.

In brief, in this invention we are recommending a process to carry out structural studies of viral and subviral pathogens, which most important characteristics are the general application, the sensitivity, the specificity, the simplicity in execution and the automatization potential.

### PREFERRED EMBODIMENT OF THE INVENTION

In a more precise way and in order to bring into practice the process of structural analysis of the viral and subviral pathogen genome that are recommended, in the first place the immobilization of the macromolecular structures are carried out which are related to the pathogen on a solid phase for which a biological sample, which previously has been homogenized in a basic pH buffer is placed in contact with the solid phase used for the immobilization, incubating the whole preparation at higher temperature than 0°C and lower than 50°C during a period greater than 15 minutes and washing the whole preparation few times with a saline solution containing a detergent and buffered to a pH near neutral.

For the immobilization of macromolecular structures related to the pathogen on the solid phase with the help of retained molecules on such phase and with affinity for such macromolecular structures; in the first place the retention of the molecules is produced, for which the solid phase is placed in contact with a solution of such molecules in a buffer at basic pH, the whole preparation is incubated at temperatures **over 4°C and lower than 50°C during a period of time greater than 15 minutes** and the preparation is washed several times with a saline solution containing a detergent and buffered with a pH near neutral. Immediately after the immobilized molecule on the solid phase is put in contact with the samples to be analyzed and the whole preparation is incubated at a temperature lower than 50°C during a period of time greater than 15 minutes, followed by several washes with the described saline solution.

In the case of viral pathogens with ADN genome, the resulting products of the immobilization are placed in contact with the necessary regents for the enzymatic amplification and the whole preparation is submitted to a series of denaturalization cycles, ringing and synthesis. The temperature and duration of each cycle depend on the sequence of nucleotides and size of the fragment to amplify.

If there is the case of viral pathogens with RNA genome or of subviral pathogens, previously to the amplification the complementary ADN is synthesized (cADN) at the genome fragment to be amplified, for which the resulting products of the immunocapture are placed in contact with the necessary regents for the cADN synthesis and the synthesis is directly made, without any previous treatment of the whole preparation, for which such preparations are incubated at a temperature not lower than 15°C, continuing with the amplification phase in the above described manner.

The final phase of the process object of the present invention consists in making an analysis of the existing structural polymorphisms in the reaction products resulting from the enzymatic amplification.

With the purpose of facilitating to a maximum the comprehension of the process that is proposed by the invention, two tables and two graphic representations of an electrophoresis gels are enclosed to this document.

**Table 1.-** Shows the homology between sequences of homologous regions of the isolate genome Type 1, that is described in the Example 1 and of the viruses JGMV, MDMV, SCMV and SrMV.

**Table 2.-** Shows the homology between the ends 3' of the UTRs of the Types 2 and 3 with the ones of the viruses JGMV, MDMV and SrMV. The isolates correspond to the ones mentioned in the Example 1 that are explained as follows.

**The graphic 3.-** Shows the structural polymorphisms, established by analysis of the restriction polymorphisms, of the TSWV isolated genomes, referred in the Example 2.

**The graphic 4.-** Shows the structural polymorphisms, established by analysis of the single chain conforming polymorphisms of the TSWV isolated genomes, referred in the Example 3.

Following are shown a series of Examples of the practical aspects of the process for the genome structural analysis of viral and subviral pathogens which constitute the object of the present invention:

### EXAMPLE 1

In this example it is described the preferential application of the process recommended in the present invention to carry out the structural analysis of the genome of three viruses belonging to the Potyvirus group which infect maize presenting different symptomatology. It is combined in this application the preferential use of antibodies for the pathogen immobilization on a solid phase with the use, also preferential, of a plate for microtitration as solid phase; making the structural analysis preferentially through the establishment and final analysis of the nucleotides sequences of the amplified fragments of the pathogen genome.

The potyvirus belong to the group of vegetable viruses which causes the greatest economic losses. The virions of the members of this group are large and flexuous particles formed by approximately 2000 copies of only one protein of the capsid, from 30-35 kD, and only one copy of RNA molecule, of approximately 10 kb. (Mathews R.E.F., 1991. Plant Virology. Academic Press). There have been described many different strains of potyvirus limited to gramineous, classified initially as virus strains of the sugarcane mosaic (sugarcane Mosaic virus (SCMV) (Pirone T.P., 1972. Sugarcane Mosaic Virus. CMI/AAB Descriptions of Plant Viruses, n° 88). The use of specific polyclonal antibodies of the virus, directed against the amino ends of the protein of the capsid, has established the grouping of the strains in four different viruses; Johnsongrass mosaic virus (JGMV), maize dwarf mosaic virus, (MDMV), sugarcane mosaic virus (SCMV), and sorghum mosaic virus (SrMV) (Shukla et al., 1989, Phytopathology, 79, 223-229). During the years 1989-1992 we carried out an epidemiologic study of the maize viruses in the main areas dedicated to this crop in Spain (Sánchez, F. et al., 1993) Investigación Agraria: Serie Protección de los Vegetales 8(2), 265-273), of all the samples three isolates were selected which were predominantly present, denominated Type 1, 2 and 3 respectively, which had very different symptomatologies, they presented very different behaviour patterns in front of a battery of indicating plants and nevertheless by ELISA and using an specific monoclonal antibody against potyvirus (Jordan, J. and Hammond, J. 1991. J. Gen. Virol. 72(1), 25-36) all of them resulted positive to potyvirus. In this Example the application of the recommended process is described in the present invention to carry out the structural analysis of the genome of these three isolates with the object of establishing a molecular typification thereof. The specific experimental details of this definite application of the process object of the present invention are detailed as follows:

Samples of infected plant leaves are individually homogenized in the proportion 1/10 (weight/volume), with a buffer Tris-HCl 0.5 M pH 8.0, containing 2% of polyvinylpirolidone, 1% of polyethilenglicol 6000, 0.... NaCL, 0.005% Tween 20 and 0.02% sodic azide. Aliquots of 50 microliters of the homogenized portions are dispersed in the small wells of a microtitration plate previously covered with a specific antiserum against potyvirus (Jordan, J. and Hammond, J. 1991. J. Gen. Virol. 72(1), 25-36) following the process described by Clark, M.F. and Bar-Joseph, M. (1984). Methods in Virology 7, 51-85. The whole preparation is incubated at 4°C during 5 hours and later the plates are washed thoroughly with the buffer PBS containing Tween 20 at 0.005%.

The inverse transcription is carried out in the same plate adding to each small well 20 microliters of buffer Tris-HCl 0.05 M pH 8.3, 0.075 M KCl, 0.003 M MgCl₂, 20mM in each dNTP, 200 units of M-MLV inverse transcriptase and 1 micromolar in the feeder 3' oligonucleotide. The feeders 3' specifically for each virus are the ones used, which nucleotides sequences are:
For JGMV: 5'-TAA GAG ACT ACG TCG TGA GAA GCT - 3'
For MDMV: 5'-CTC TCA CCA CGA AAC TCG C - 3'
For SCMV: 5'-CCC AGT GCA GTC CAT CTC - 3'
For SrMV: 5'-CAA TAC TCG CAA CTC CTG - 3'.

After incubating at 37° C during 1 hour, it is added to each small well 80 microliters of composition amplification mixture and of final concentrations: Buffer Tris-HVl 60 mM pH 9, 0.015 mM KC1, 2.1 mM MgC12, 20 mM in each dNTP, 0.005% of bovine serum albumin (BSA) and 1 micromolar of feeder 5' which sequence of nucleotides is: 5'-ATA CAG ARM GNC ACA CAG C - 3', where R = A o G, M = A o C and N = A, C, G or T.

The feeders were designed comparing sequences published of different potyvirus capable of infecting maize. A feeder 5' was selected which could ring in a reserved area between the different maize potyvirus. To achieve the consensus several positions were degenerated. The feeder 3' was selected to be specific for each one of the viruses.

The plates were heated at 94°C during two minutes, they were cooled off until they reached 72° C, and 1.6 units of Taq ADN polymerase were added and were submitted to thirty cycles of heating and cooling. Each cycle was formed by the following phases: forming of rings during one minute at 52° C, elongation during one minute at 72° C and denaturalization during 30 s. at 93° C. In the final cycle the time of elongation was increased to five minutes. The thermic gradients are of 0.3° C s⁻¹. Immediately after one aliquot part of the mixture of amplification is taken and later it is established, directly and without previous molecular clonation, the sequence of nucleotides of the amplified product for which the procedure described by Bachman, B. et al. 1990. Nucleic Acid Research 18, 1309 was followed.

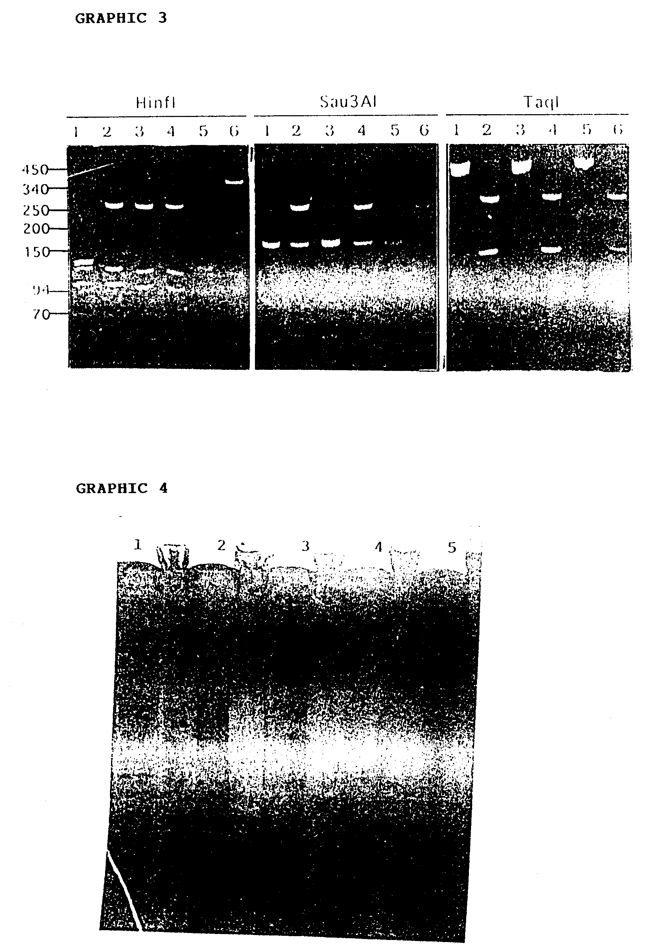

In the case of the isolate Type 1, the homology between the nucleotides sequence of the amplified fragment and the published one for the sugarcane mosaic virus, (SCMV) permits to classify such Type 1 as SCMV **(Figure 1).** In the case of the isolates Type 2 and Type 3 the analysis of the sequences of the amplified fragments allows to classify them as two different strains of the maize dwarf mosaic virus, MDMV **(Figure 2).**

It was possible in this way to classify, in a simple way, the three potyvirus which were found principally in the Spanish maize crops, starting from a situation in which it was known only the fact that they were potyvirus and that they behave differently compared with the indicating plants.

### EXAMPLE 2

In this example it is described the preferential application of the recommended process in the present invention to carry out the structural analysis of the genome of six isolates, designated as 3, 4, 7, 8, B3 and B4, of a Tospovirus, the tomato sppoted wilt virus, (TSWV), that by immunologic techniques had been classified as belonging to the same serotype. In this application it is combined the preferential use of specific antibodies for the immobilization of the pathogen on solid phase with the use, also preferential, of a microtitration plate as solid phase; making the structural analysis preferentially by a restriction analysis of the genome fragments of the amplified pathogen. The tospovirus are virus that present some of the characteristics of the animal Bunyavirus, as for example having a lipidic cover, genome formed by three molecules of a single chain RNA, homology of sequences and cytoplasmatic maturity of its particles, (Elliot, R.N. (1990). J. of Gen. Virol. 71: 501-552). A representative member of the group is the tomato sppoted wilt virus, (TSWV), the pathogen that infects a great number of horticultural and ornamental plants in the whole world with devastating effects. The specific experimental details of this definite application of the process object of the present invention are detailed as follows:

Samples of plant leaves infected with TSWV are individually homogenized in the proportion 1/10 (weight/volume), with a buffer Tris-HCL 0.5 M pH 8.0, containing 2% polyvinylpirolidone, 1% polyethilenglicol 6000, 0.8% NaCl, 0.005% Tween 20 and 0.02% sodic azide. Aliquots of 50 microliters of the homogenized are dispersed in the small wells of a microtritration plate previous covered with antiserum against TSWV following the process described by Clark, M.F. and Bar-Joseph, M (1984). Methods in Virology 7, 51-85. The whole preparation is incubated a 4° C during 5 hours and later the plates are thoroughly washed with a buffer PBS containing Tween 20 at 0.005%. The inverse transcription is carried out in the same plate adding to each small well 20 microliters of buffer Tris-HCl 0.05 M ph 8,3, 0.075 M KCl, 0.003 M MgCl₂, 20 mM in each dNTP, 200 units of M-MLV inverse transcriptase and 1 micromolar in the feeder 3' oligonucleotide which sequence of nucleotides is: 5'-CAT GGA TCC TGC AGA GCA ATT GTG TCA - 3'. After incubating at 37° C during 1 hour, it is added to a small well 80 microliters of composition amplification mixture and of final concentrations: Buffer Tris-HCl 60 mM pH 9, 0.015 mM KCl, 2.1 mM MgCl2, 20 mM in each dNTP, 0.005% of bovine serum albumin (BSA) and 1 micromolar of feeder 5', which sequence of nucleotides is; 5'-ATC AAG CTT CTG AAG GTC AT - 3'. The plates are heated at 94° C during two minutes, they are cooled off until 72° C. and 1.6 units of Taq ADN polymerase were added during thirty cycles of heating and cooling. Each cycle is formed by the following phases: forming of rings during one minute at 52° C, elongation during one minute at 72° C and denaturalization during 30 s. at 93° C. In the final cycle the elongation time was increased to five minutes. The thermic gradients are of 0.3° C. s⁻¹. Immediately after three aliquot parts of the amplification products are taken and they are individually submitted to a restriction by the enzymes HinfI, Sau3aI and TaqI, following the recommended protocols by the manufacturer, and later continuing with the electrophoretic fractioning of the products of restriction and the subsequent analysis of such fractioning. In **Figure** **3**, the existing polymorphisms are shown. The advantage of this way of typification is to allow the differentiation, of a fast and precise way, of isolates that serologically are undistinguishable.

### EXAMPLE 3

In this Example it is described the preferential application of the recommended process in the present invention to carry out the structural analysis of the genome of three isolates of the tomato sppoted wilt virus (TSWV), the ones denominated as 7, B3 and B4. In this application it is combined the preferential use of antibodies for the immobilization of pathogen on solid phase with the use, also preferential, of a microtritration plate as solid phase; making the structural study preferentially by an analysis of single chain conforming polymorphisms (SSCP) of the amplification products. The experimental details of the immobilization phases, inverse transcription and enzymatic amplification are the same as the ones described in the Example 2 of the present document. In the present preferential application of the process object of the present invention, once the amplification cycles are finished, 5 microliters of the reaction mixture are taken, 10 microliters of formamide are added, the preparation is heated at 94° C during five minutes, and then cooled off quickly over ice and the fractioning is made of the different conform-meters according to the described protocol by Oto, M. et al. (1993). Anal. Biochem 213,19-22. In the graphic 4, the obtained results are shown. In this graphic 1, 2, 3 and 4 respectively represent the isolates 7, 8, B3 and 5 represent ADN of the fago lambda cut with the enzyme of HIndIII restriction.

**TABLE 1.**

| Homology between sequences of homologous regions of the genomes of isolate Type 1, referred in the Example 1, and of the viruses JGMV, MDMV, SCMV AND SrMV. | | | | |
|---|---|---|---|---|
| REGION OF THE GENOME | JGMV | MDMV | SCMV | SrMV |
| 3' UTR + capsid | 41.6% | 58.8% | 81.7%-79% | 54.8% |
| UTR | 72% | 66% | 84%-87% | 62% |

**TABLE 2.**

| Homology between the UTRs of the isolates Type 2 and 3, with the ones of the viruses JGMV, MDMV, SCMV and SrMV. The isolates correspond to the ones referred in Example 2. | | | | |
|---|---|---|---|---|
| ISOLATE | JGMV | MDMV | SCMV | SrMV |
| Type 2 | 76% | 96% | 63% | 66% |
| Type 3 | 75% | 95% | 64% | 64% |

## Claims

**1.-** Process to structurally analyze the genome of viral and subviral pathogens, establishing in it the following phases or operative phases:
a) Immobilization on the solid phase of macromolecular structures related to the pathogen.
b) Synthesis of the complementary ADN (cADN) to a fragment of the pathogen genome.
c) Enzymatic amplification of the cADN or of a genome fragment of ADN virus.
Characterizing such process because the final phase consists in making an analysis of the existing structural polymorphisms in the resulting reaction products of the enzymatic amplification.

**2.-** Process to structurally analyze the genome of viral and subviral pathogens, according to claim 1, which is characterized in that the solid phase used for the immobilization of the macromolecular structures related to the pathogen is a microtritration plate.

**3.-** Process to structurally analyze the genome of viral and subviral pathogens according the claim 1, characterized in that the immobilization on the solid phase of macromolecular structures related to the pathogen is carried out by the help of retained molecules on such solid phase and which have affinity with such macromolecular structures.

**4.-** Process to structurally analyze the genome of viral and subviral pathogens according to claim 3, characterized in that the solid phase is a microtritration plate.

**5.-** Process to structurally analyze the genome of viral and subviral pathogens, according to claim 3, characterized in that the molecules are antibodies.

**6.-** Process to structurally analyze the genome of viral and subviral pathogens, according to claim 5, characterized in that the solid phase is a microtritration plate.
